# EUROPÄISCHE PATENTSCHRIFT

(11) **EP 1 332 140 B1**
(45) Veröffentlichungstag und Bekanntmachung des Hinweises auf die Patenterteilung: **27.02.2008**
(21) Anmeldenummer: 01988715.7
(22) Anmeldetag: 12.10.2001
(51) Int. Cl.: C07D 307/83

(54) **VERFAHREN ZUR HERSTELLUNG VON MINTLACTON**
METHOD FOR THE PRODUCTION OF MENTHALACTONE
PROCEDE DE PRODUCTION DE LACTONE DE MENTHE

(30) Priorität: 25.10.2000 DE 10052803
(43) Veröffentlichungstag der Anmeldung: 06.08.2003
(73) Patentinhaber: Symrise GmbH & Co. KG, 37603 Holzminden (DE)
(72) Erfinder: KOCH, Oskar, 37079 Göttingen (DE); KUHN, Walter, 37603 Holzminden (DE)
(74) Vertreter: Eisenführ, Speiser & Partner
(86) Internationale Anmeldenummer: PCT/EP2001/011813
(87) Internationale Veröffentlichungsnummer: WO 2002/034737

(56) Entgegenhaltungen:
- US-A- 4 407 740
- FOOTE, CHRISTOPHER S. ET AL: "Photosensitized oxygenation of alkyl-substituted furans" TETRAHEDRON,(1967), 23(6), 2583-99, XP001061562
- FOOTE, CHRISTOPHER S. ET AL: "Photosensitized oxygenation of alkyl-substituted furans" TETRAHEDRON,(1967), 23(6), 2601-2608, XP002155155 in der Anmeldung erwähnt
- TANYELI, CIHANGIR ET AL: "A facile synthesis of (.+-.)-mintlactone" SYNTH. COMMUN. (1997), 27(19), 3471-3476 , XP001061557
- TSUBOI, SADAO ET AL: "New synthesis of (.+-.)-menthofuran" J. ORG. CHEM. (1980), 45(8), 1517-20 , XP001061503
- HIRSCH, JERRY A. ET AL: "Hydrolysis of.alpha.,.alpha.-dimethoxydihydromenthofu ran" J. ORG. CHEM. (1967), 32(9), 2915-16 , XP001061502
- PATENT ABSTRACTS OF JAPAN vol. 018, no. 075 (C-1163), 8. Februar 1994 (1994-02-08) & JP 05 286961 A (TOYOTAMA KORYO KK), 2. November 1993 (1993-11-02)
- YOSHIDA TOSHIO: AGRIC. BIOL. CHEM., Bd. 44, Nr. 7, 1980, Seiten 1535-1543, XP001061594 in der Anmeldung erwähnt

## Beschreibung

Die vorliegende Erfindung betrifft ein Verfahren zur Herstellung von 3,6-Dimethyl-5,6,7,7a-tetrahydro-4H-benzofuran-2-on ("Mintlacton") aus Hydroxymenthofurolacton. Mintlacton ist ein wichtiger Inhaltsstoff der Pfefferminzpflanze und daher für die Herstellung naturidentischer Mintaromen essentiell.

Die Synthese von Mintlacton (II) erfolgt üblicherweise aus Hydroxymenthofurolacton (I) durch Umsetzung mit dem Reduktionsmittel Natriumborhydrid.

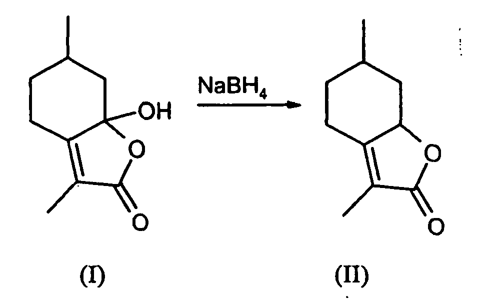

Das Verfahren in der Reduktionsstufe ist jedoch aufgrund des hohen Preises des Reduktionsmittels Natriumboranat unwirtschaftlich und aufgrund des bei dem Prozess entstehenden Wasserstoffs müssen entsprechende Sicherheitsmaßnahmen getroffen werden. Aus diesem Grund wird die Dosierung des Reduktionsmittels über einen weiten Zeitraum von 7 Stunden ausgedehnt, um den Gehalt an elementaren Wasserstoff in der Abluft möglichst niedrig zu halten. Dies hat zwangsläufig lange Reaktionszeiten sowie hohe Kosten zur Folge.

Es ist daher wünschenswert, ein besseres und kostengünstigeres Verfahren zur Herstellung von Mintlacton anzuwenden.

Eine mögliche Alternative zu dem oben genannten Verfahren stellt die Hydrierung von Dehydromenthofurolacton (III) dar, was im Labormaßstab in Gegenwart von Platindioxid als Katalysator in Journal of. Agric. Biol. Chem. 44(7), 1535(1980) beschrieben wird. Die Herstellung des Dehydromenthofurolactons (III) erfolgt durch säurekatalysierte Wasserabspaltung aus dem Hydroxymenthofurolacton (I).

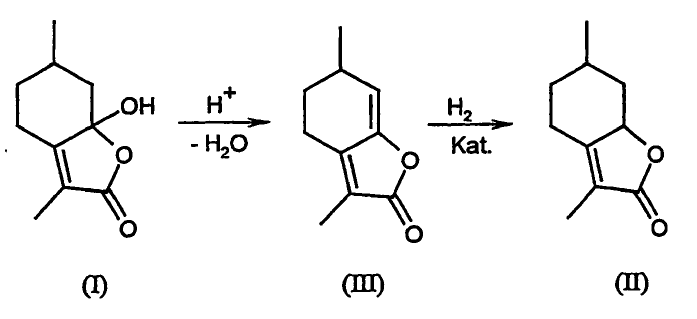

Die dort angegebenen Ausbeuten an Mintlacton liegen unter 50% und waren damit unwirtschaftlich.

Weiterhin ist aus Tetrahedron 23, 2601 (1967) bekannt, dass bei der Hydrierung von Hydroxymenthofurolacton (I) mit einem Palladium-Katalysator in Ethanol ein Isomerengemisch der nachstehenden Ketosäuren IV entsteht. Die Umsetzung ist eben falls nur im Labormaßstab durchgeführt worden.

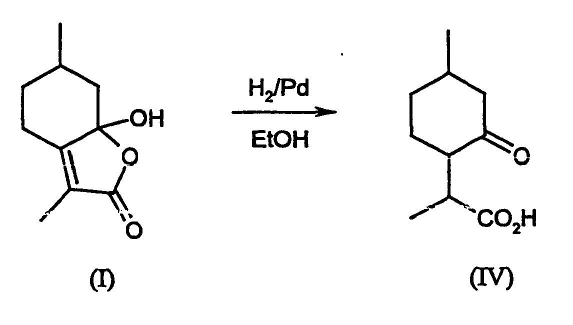

Weiterhin ist in Tetrahedron 23, 2601 (1967) beschrieben, dass eine Behandlung dieser Ketonsäuren IV mit Phosphorpentoxid oder Kaliumbisulfat zu dem zum Mintlacton isomeren ungesättigten Lacton V führt.

Weiterhin ist in Tetrahedron 23, 2601 (1967) beschrieben, dass eine Behandlung dieser Ketonsäuren IV mit Phosphorpentoxid oder Kaliumbisulfat zu dem zum Mintlacton isomeren ungesättigten Lacton V führt.

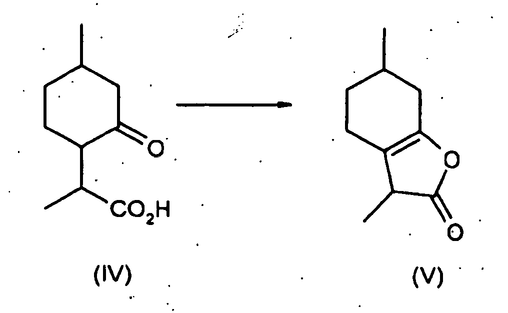

Die Löslichkeit des Hydroxymenthofurolactons (I) in Ethanol wie auch in anderen organischen Lösungsmitteln ist äußerst schlecht und somit eine technische Hydrierung wie in Tetrahedron 23, 2601 (1967) beschrieben, nicht praktikabel.

FOOTE, Christopher S. et al. offenbaren in TETRAHEDRON, 1967, 23(6), 2583-99 unter anderem ein Verfahren zur Herstellung von Mintlacton.

US-A-4 407 740 (KOEPSEL Manfred et al.) offenbart unter anderem die Umsetzung von Hydroxymenthofurolacton mit Natriumborhydrid zu Mintlacton.

Es wurde ein Verfahren zur Herstellung von Mintlacton durch Hydrierung von Hydroxymenthofurolacton zum Zwischenprodukt Dihydrohydroxymenthofurolacton und nachfolgender Wasserabspaltung gefunden.

Das erfindungsgemäße Verfahren kann durch die folgende Reaktionsgleichung beschrieben werden:

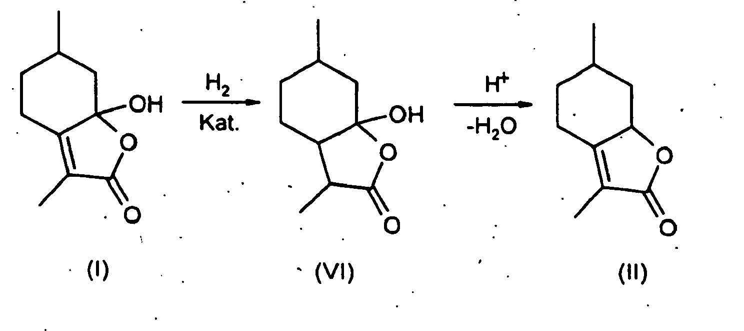

Es ist außerdem überraschend, dass das nach Hydrierung entstehende Zwischenprodukt VI (Gleichgewichtsmischung zwischen Verbindungen IV und VI) durch säurekatalysierte Wasserabspaltung nicht zu dem Produkt V, sondern in ebenfalls sehr guten Ausbeuten zum Mintlacton (II) führt.

Vorteilhaft ist außerdem, dass eine Aufarbeitung der Zwischenstufe (IV/VI), wie sie in der Regel durch Destillation oder Kristallisation erfolgt, nicht nötig ist. Die aus der Hydrierung vorliegende alkalisch-wässrige Lösung wird mit Hilfe von Mineralsäuren angesäuert und mit einem nicht wassermischbaren organischen Lösungsmittel, z.B. Toluol extrahiert. Durch Zugabe katalytischer Mengen einer Mineralsäure zu dieser organischen Phase wird azeotrop das abgespaltene Wasser entfernt. Aus diesem Grund sind solche nicht wassermischbaren organischen Lösungsmittel bevorzugt, die gleichzeitig ein Azeotrop mit Wasser bilden.

Zur Hydrierung nach dem erfindungsgemäßen Verfahren wird das Hydroxymenthofurolacton in einer wässrigen Lauge gelöst.

Als wässrige Laugen seien beispielsweise genannt: wässrige Alkalihydroxid-Lösungen wie Lithium-, Natrium- oder Kaliumhydroxid.

Die Konzentration der wässrigen Lauge liegt im allgemeinen im Konzentrationsbereich von 1 bis 80 Gew.-%, bevorzugt im Bereich von 2 bis 20 Gew.-%.

Die Konzentration des Hydroxymenthofurolacton in der wässrigen Lauge kann in weiten Grenzen schwanken. Konzentration von 10 bis 60, vorzugsweise 20 bis 50 Gew.-% werden bevorzugt.

Für die Hydrierung von Hydroxymenthofurolacton (I) geeignete Katalysatoren umfassen die üblichen Hydrierkatalysatoren - also Edelmetalle wie Platin, Palladium, Rhodium, andere Übergangsmetalle wie Molybdän, Wolfram, Chrom, Eisen, Kobalt, Nickel, jeweils einzeln oder im Gemisch.

Der Hydrierkatalysator wird vorzugsweise aus Platin, Palladium und deren Verbindungen gewählt. Er kann in Form der Metalle beispielsweise fein verteilt als Platin-Schwarz oder Palladium-Schwarz oder in Form von Verbindungen dieser Metalle, z.B. als Metallsalz oder Metallkomplex, eingesetzt werden. Alle Einsatzformen des Katalysators können auch auf Trägern aufgebracht sein.

Bevorzugte Katalysatoren umfassen z.B. Platin-Verbindungen PtO₂, H₂PtCl₆, PtCl₂, PtCl₄, PtBr₂, PtJ₂, Pt(NH₃)NO₂)₂, Pt(NH₃)₄Cl₂, Pt(H₂NCH₂CH₂CH₂)₂Cl₂, und die Palladium-Verbindungen PdO, PdSO₄, PdBr₂, PdCl₂, Pd(CH₃CO₂)₂ Pd(NH₃)₄(NO₃)₂, Pd-(II)-acetylacetonat und Pd-(II)-trifluoracetat.

Als Katalysatorträger eignen sich alle technisch üblichen Katalysatorträger, z.B. solche auf Basis von Kohle, Elementoxiden, Elementcarbiden oder Elementsalzen in verschiedenen Anwendungsformen. Beispiele für Elementoxid-Katalysatorträger sind Siliciumdioxid (natürliche oder synthetische Kieselsäuren, Quarz), Aluminiumoxid, Tonerden, natürliche und synthetische Alumosilikate (Zeolithe), Titandioxid (Rutil, Anatas), Zirkonoxid oder Zinkoxid. Bevorzugte Elementcarbide und -salze umfassen Siliciumcarbid, Aluminiumphosphat, Bariumsulfat, Calciumcarbonat u.a. Sie können sowohl im Sinne chemisch einheitlicher Reinsubstanzen als auch im Gemisch eingesetzt werden. Erfindungsgemäß als Katalysatorträger eignen sich sowohl stückige als auch pulverförmige Materialien.

Der Einsatz der Katalysatoren auf Trägern wird bevorzugt, besonders bevorzugt ist der Einsatz von Platin und Palladium auf Trägern, z.B. Kohle.

Die Beladung des Trägers mit Katalysator beträgt vorzugsweise 0,1 bis 15, insbesondere 5 bis 10 Gew.%, bezogen auf die Summe von Träger und Katalysator, berechnet als Metall.

Der Katalysator wird in Mengen von 0,001 bis 5, vorzugsweise 0,01 bis 1 Gew.-%, berechnet als Metall und bezogen auf Hydroxymenthofurolacton (I), eingesetzt.

Die Hydrierung von Hydroxymenthofurolacton (I) kann mit Wasserstoff unter einem Druck von 1 bis 100, vorzugsweise 10 bis 20 bar und bei Temperaturen von 5 bis 200, vorzugsweise 20-40°C erfolgen.

Die säurekatalysierten Wasserabspaltung nach dem erfindungsgemäßen Verfahren wird im allgemeinen in Gegenwart von Mineralsäuren durchgeführt.

In einer bevorzugten Ausführungsform führt man hierzu eine Extraktion aus der wässrigen Hydrierlösung mit einem nicht wassermischbaren Lösungsmittel durch, das gleichzeitig mit Wasser ein Azeotrop bildet, wie z.B. Toluol oder Xylol. Vorzugsweise wird bei dieser Extraktion ein pH-Wert von etwa 1 eingestellt. Dazu sind Mineralsäuren wie z.B. Salzsäure, Schwefelsäure oder Phosphorsäure geeignet.

Nach diesem Verfahren erhält man Mintlacton in hoher Reinheit.

### Beispiel

400g (2,20 Mol) Hydroxymenthofurolacton, gelöst in 1.000 g Natronlauge, 10-proz. und 2,0g Pd/C werden in einen Autoklaven gegeben und bei 20 bar und einer Temperatur von 25-30°C hydriert. Die Reaktion ist nach 2 h beendet.

Nach Abkühlen auf Raumtemperatur und Abtrennen des Katalysators wird der pH-Wert des Filtrats durch Zugabe von 570g Schwefelsäure, 30-proz. von 13 auf 1 gestellt Die sich dabei absetzende Ölphase extrahiert man mit 600g Toluol. Nach Phasentrennung wird mit 5g Schwefelsäure, konz. versetzt und 3 h am Wasserabscheider auf Rückfluss gekocht, wobei sich ca. 60g Wasser abscheiden. Nach Abkühlen auf RT wird die org. Phase nochmals mit 200g Wasser gewaschen und dann an einer 15cm-Vigreux-Kolonne destilliert. Man erhält 334g Mintlacton mit einer Reinheit von 99%. Die Ausbeute über beide Stufen beträgt 91% der Theorie.

## Patentansprüche

1. Verfahren zur Herstellung von Mintlacton durch Hydrierung von Hydroxymenthofurolacton zum Zwischenprodukt Dihydrohydroxymenthofurolacton und nachfolgender Wasserabspaltung.

2. Verfahren nach Anspruch 1, wonach die Hydrierung in wässrig-alkalischer Lösung bei einem pH-Wert von 8-14 stattfindet

3. Verfahren nach Anspruch 1, wonach die Hydrierung bei einem Wasserstoffdruck von 1-100 bar stattfindet.

4. Verfahren nach Anspruch 1, wonach die Hydrierung bei einer Temperatur von 5-200°C stattfindet.

5. Verfahren nach Anspruch 1, wonach die Hydrierung in Gegenwart von Platin oder Palladium als Katalysator stattfindet.

6. Verfahren nach Anspruch 1, wonach das Zwischenprodukt nach der Hydrierung mit einem nicht wassermischbaren organischen Lösungsmittel, das gleichzeitig ein Azeotrop mit Wasser bildet, extrahiert wird.

## Claims

1. A process for preparing mint lactone by hydrogenating hydroxymenthofurolactone to give the intermediate dihydrohydroxymenthofurolactone and subsequent elimination of the water.

2. A process according to claim 1, wherein hydrogenation takes place in an aqueous alkaline solution at a pH of 8-14.

3. A process according to claim 1, wherein hydrogenation takes place at a hydrogen pressure of 1-100 bar.

4. A process according to claim 1, wherein hydrogenation takes place at a temperature of 5-200°C.

5. A process according to claim 1, wherein hydrogenation takes place in the presence of platinum or palladium as catalyst.

6. A process according to claim 1, wherein the intermediate, after the hydrogenation, is extracted with a water-immiscible organic solvent which simultaneously forms an azeotrope with water.

## Revendications

1. Procédé pour la préparation de lactone de menthe par hydrogénation d'hydroxymenthofurolactone en un produit intermédiaire de dihydrohydroxymenthofurolactone et par élimination subséquente d'eau.

2. Procédé selon la revendication 1, selon lequel l'hydrogénation a lieu dans une solution aqueuse-alcaline à une valeur de pH de 8-14.

3. Procédé selon la revendication 1, selon lequel l'hydrogénation a lieu à une pression d'hydrogène de 1-100 bar.

4. Procédé selon la revendication 1, selon lequel l'hydrogénation a lieu à une température de 5-200°C.

5. Procédé selon la revendication 1, selon lequel l'hydrogénation a lieu en présence de platine ou de palladium comme catalyseur.

6. Procédé selon la revendication 1, selon lequel le produit intermédiaire est extrait après l'hydrogénation avec un solvant organique non miscible à l'eau, lequel forme simultanément un azéotrope avec l'eau.
